# EUROPEAN PATENT SPECIFICATION

(11) **EP 2 421 447 B1**
(45) Date of publication and mention of the grant of the patent: **19.10.2016**
(21) Application number: 10719765.9
(22) Date of filing: 19.04.2010
(51) Int. Cl.: A61B 17/16, A61B 17/32

(54) **IMPROVED BONE RESECTOR**
VERBESSERTER KNOCHENRESEKTOR
INSTRUMENT AMÉLIORÉ DE RÉSECTION OSSEUSE

(30) Priority: 23.04.2009 GB 0906930
(43) Date of publication of application: 29.02.2012
(60) Divisional application: 14188906.3
(73) Proprietor: Orthosonics Limited, Maidenhead Berkshire SL6 7BU (GB)
(72) Inventor: YOUNG, Michael John Radley, Ashburton South Devon TQ13 7JX (GB)
(74) Representative: Dauncey, Mark Peter
(86) International application number: PCT/GB2010/000776
(87) International publication number: WO 2010/122288

(56) References cited:
- EP-A1- 1 110 509
- WO-A1-2006/059120
- WO-A2-2008/089174
- US-A- 3 642 002
- US-A- 4 188 952
- US-A1- 2002 099 400
- US-A1- 2003 187 383
- US-A1- 2007 260 253

## Description

The present invention relates to a surgical tool for cutting both cortical and cancellous bone. More particularly but not exclusively, it relates to a surgical tool for bone resection in minimal access surgical techniques.

It is known, for example from our British patent application No. GB2420979A, to cut both cortical and cancellous bone in the course of surgical procedures, using ultrasonically activated instruments having a cutting edge with a saw-tooth profile.

In many situations, conventional powered oscillating saws having sharp tooth profiles and lateral tooth offsets are also effective. However, joint replacement procedures (amongst others) are increasingly often being carried out through incisions of reduced dimensions, to reduce soft tissue trauma. While this has clear benefits in respect of post-operative healing, it places greater demands on the surgeon's skill and dexterity to achieve the correct bone facet geometry for implant location, working through such restricted incisions. The use of such minimally invasive techniques can thus paradoxically produce increased risks of significant collateral damage to sensitive tissue structures adjacent the desired operative site. A conventional sharp-toothed powered saw can readily cut ligaments, vascular and nerve tissue with only transient contact with its sharp cutting edges.

Ultrasonically-vibrated blades need not be as sharp, cutting only when activated. They are also tunable to transmit energy selectively into hard, bony matter in preference to soft tissue. They hence tend to cause less accidental trauma. Unfortunately, such tools currently perform their prime function of cutting bone significantly more slowly than conventional oscillating saws, and so have not been as widely adopted as had been expected, particularly when their greater complexity and cost is taken into account.

A further issue that has been encountered is that ultrasonically-vibrated osteotomes can lead to localised heating as ultrasonic energy is dissipated into the bone. This may lead to localised bone necrosis and consequent poor healing.

A further problem with conventional oscillating saws is that a portion of the oscillatory motion tends to be transmitted from the tool into the surgeon's hand. This low-frequency vibration can be uncomfortable, may lead to more rapid fatigue in the surgeon's hand and fingers, and with prolonged exposure might even result in problems such as "white finger".

It is hence an object of the present invention to provide improved surgical bone-cutting tools that obviate at least some of the above problems, while allowing rapid and accurate bone resection with minimal damage to adjacent soft tissues or to the remaining bone.

According to a first aspect of the present invention, there is provided a surgical tool adapted to cut osseous material comprising cutting head means having elongate cutting edge means, said cutting head means being operatively connected both to means to generate ultrasonic vibrations and to means to displace the cutting head means reciprocally. The invention is decribed in claim 1, and the preferred embodiments in the dependent claims.

The reciprocal displacement means preferably acts generally parallely to the cutting edge means.

Preferably, the reciprocal displacement means is adapted to produce an oscillatory motion at a frequency of 250Hz or lower.

Advantageously, such oscillatory frequency is at least 20Hz.

Optionally, said oscillatory frequency is between 40 and 60Hz, for example being at approximately 50Hz.

Preferably, said means to generate ultrasonic vibrations is adapted to generate said vibrations at a frequency of at least 20kHz.

Advantageously, said ultrasonic vibrations are generated at a frequency of 60kHz or below.

Optionally, said ultrasonic vibrations are generated at a frequency of approximately 40kHz.

Preferably, the relative amplitudes of the ultrasonic vibrations and the oscillatory motion of the cutting head are such that a peak velocity of the cutting head due to the ultrasonic vibrations is greater than a peak velocity resulting from the oscillatory motion.

Advantageously, the peak cutting head velocity due to the ultrasonic vibrations is at least twice that resulting from the oscillatory motion.

The peak cutting head velocity due to the ultrasonic vibrations may be at least three times that resulting from the oscillatory motion.

The peak cutting head velocity due to the ultrasonic vibrations is preferably no more than ten times that resulting from the oscillatory motion.

Advantageously, the peak cutting head velocity due to the ultrasonic vibrations is no more than seven times that resulting from the oscillatory motion.

Preferably, the ultrasonic vibrations comprise longitudinal ultrasonic vibrations directed generally parallelly to the oscillatory motion and to the cutting edge means.

The cutting head means may comprise an elongate waveguide with the cutting edge means disposed adjacent a distal end thereof.

The cutting edge means may comprise an elongate array of tooth means.

Said tooth means may each comprise saw tooth means.

In a preferred embodiment, the means to displace the cutting head means reciprocally is provided with first counterweight means for the cutting head means, reciprocally displaceable out of phase with the cutting head means.

Advantageously, the first counterweight means is displaceable substantially in antiphase therewith.

A centre of mass of the cutting head means and the first counterweight means may remain substantially stationary.

Advantageously, the means to displace the cutting head means reciprocally displaces both the cutting head means and the means to generate ultrasonic vibrations.

The reciprocal displacement means may then be provided with second counterweight means for both the cutting head means and the means to generate ultrasonic vibrations, reciprocally displaceable out of phase therewith.

The second counterweight means may be displaceable substantially in antiphase therewith.

A centre of mass of the cutting head means, the means to generate ultrasonic vibrations and the second counterweight means may thus remain substantially stationary.

Preferably, the reciprocal displacement means comprises a rotatable generally cylindrical body having a first and a second track means each extending continuously around the body, with the cutting head means and optionally the means to generate ultrasonic vibrations being moveably engaged with the first track means, and the respective counterweight means being moveably engaged with the second track means.

Advantageously, each said track means comprises groove means.

The cutting head means and counterweight means may each then be provided with coupling pin means constrained to move within respective groove means.

Preferably, each track means extends around the cylindrical body at an angle to a rotational axis thereof, with the first track means being angled in an opposite sense to the second track means.

A longitudinal disposition of each track means thus varies around a circumference of the cylindrical body.

When the cylindrical body is rotated, the cutting head means and counterweight means, being coupled to respective track means, are driven to move reciprocally, and out of phase each with the other, optionally in antiphase each with the other.

Preferably, the reciprocal displacement means is provided with motor means, adapted to drive the cylindrical body rotatingly.

Advantageously, said motor means is provided with means to select a desired speed of rotation of the body.

Preferably, the tool comprises manually graspable and manipulable outer casing means, enclosing at least the reciprocal displacement means and the means to generate ultrasonic vibrations.

Advantageously, the tool comprises an elongate outer casing means having the cutting head means extending longitudinally therefrom.

In a preferred embodiment, said cutting edge means is provided with a plurality of teeth, arrayed therealong.

Each said tooth may have a hooked profile.

A tip of each said hooked tooth may extend generally towards a distal end of the tool.

Said profile may be suitable for use in any osteotome, particularly ultrasonically-vibratable osteotomes.

There is provided an exemplary method of cutting osseous material comprising the steps of providing a tool as described in the first aspect above, applying a cutting edge means thereof to a zone of osseous material to be cut, activating both the reciprocal displacement means and the means to generate ultrasonic vibrations, and guiding the tool manually until a desired cut or facet has been produced.

Preferably, the above exemplary method is adapted to cut cortical and/or cancellous bone as part of a surgical procedure.

Advantageously, the exemplary method comprises the steps of creating an incision leading from a body surface to the bone to be cut and introducing cutting head means of the tool therethrough.

The method may comprise the step of cutting bone to prepare for implantation of a prosthetic device, such as an orthopaedic joint replacement.

The method may comprise the step of cutting bone to remove an implanted prosthetic device, for example as part of a revision procedure for an orthopaedic joint replacement.

An embodiment of the present invention will now be more particularly described by way of example and with reference to the figures of the accompanying drawings, in which:
**Figure 1A** is a schematic longitudinal cross-section of an internal operative structure of a first bone resector tool embodying the present invention;
**Figure 1B** is a cross-section of a driving stud separated from the tool shown in Figure 1A;
**Figure 1C** is a scrap radial cross-section of the driving stud shown in Figure 1B, in operation within the tool shown in Figure 1A;
**Figure 1D** is a schematic longitudinal cross-section of an internal operative structure of a second bone resector tool embodying the present invention;
**Figure IE** is a scrap elevation of a cutting head of the second tool shown in Figure 1D;
**Figure 2** is a side elevation of a drive converter element separated from the tool shown in Figure 1A or the tool shown in Figure 1D;
**Figure 3** is a side elevation of a driveshaft separated from the tool shown in Figure 1A or the tool shown in Figure 1D;
**Figure 4** is a side elevation of the drive converter element shown in Figure 2, together with its drive arrangements and a counterweight cylinder coupled thereto;
**Figure 5** is a side elevation of the drive converter element shown in Figure 2, together with a blade driving cylinder coupled thereto; and
**Figure 6** is a side elevation of either of the tools shown in Figure 1A and 1D, including its outer casing in sectioned and partially disassembled form.

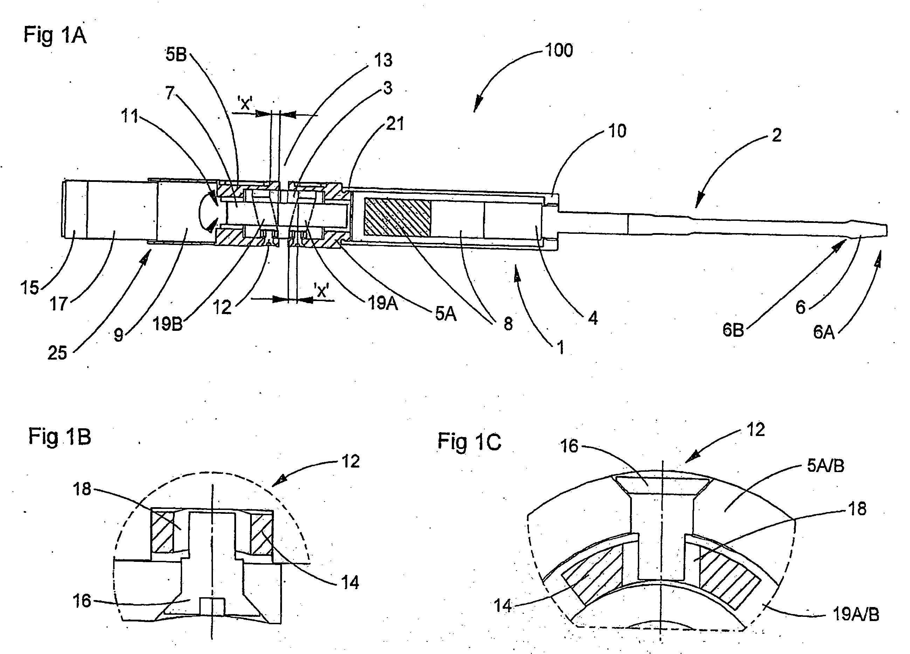

Referring now to the Figures and to Figure 1A in particular, an acoustic system 1 of a first bone resector tool 100 comprises a longitudinal mode ultrasonic transducer 8 (typically comprising a stack of piezo electric-elements) connected by a horn arrangement 4 to an elongate exchangeable blade portion 2. The blade portion 2 has a cutting head 6 at its distal end, provided with one or more lateral cutting edges. (The cutting edge(s) are not shown in detail in Figure 1A, but may typically comprise an array of saw teeth, set in a desired geometry. The present invention is believed to be suitable for use with most or all known forms of osteotome blade geometries).

The particular tool 100 shown produces ultrasonic vibrations in its blade portion 2 which have a maximum longitudinal displacement amplitude, at a distal tip 6A of the cutting head 6, of between 80 and 140µm. The ultrasonic transducer 8, horn 4 and blade portion 2 are tuned such that the distal tip 6A is at an antinode of the ultrasonic vibrations. The displacement amplitude at a proximal end 6B of the cutting head 6 will be about 60% of that at the distal tip 6A.

It is found that ultrasonic vibrations in the near ultrasonic region are suitable, for example in the range 20-60 kHz. A frequency of close to 40 kHz is currently preferred. This produces a peak blade velocity at the distal tip 6A of 10-50 m.s⁻¹

The acoustic system 1 is held within elongate cylindrical housing 10, with the blade portion 2 projecting distally therefrom. At its proximal end, the housing 10 is fastened by a screw coupling 21 to a blade driving cylinder 5A, the function of which is described below.

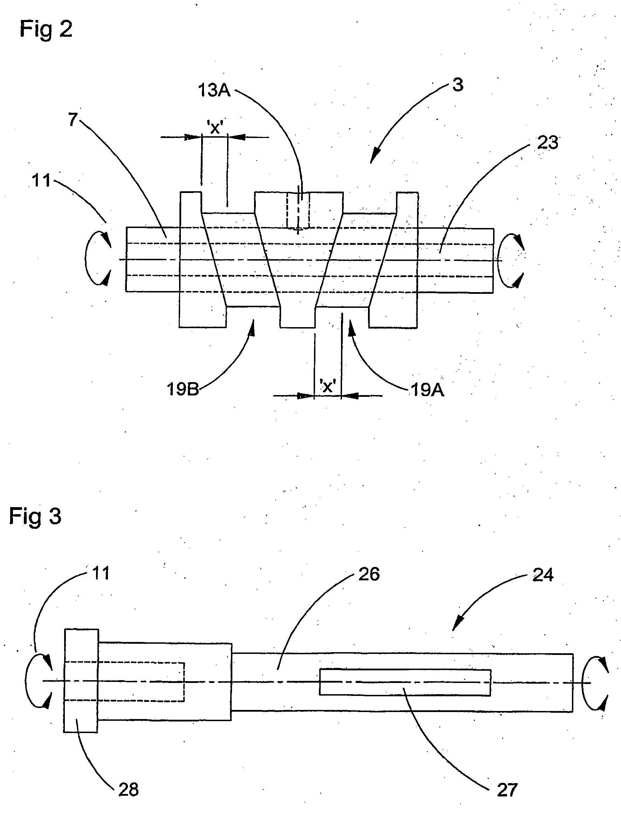

An electric motor 17, located adjacent a proximal end of the tool 100 and acting through a gearbox 9 and a driveshaft 24 (see Figure 3), drives a shaft 7 of a drive converter element 3 located generally centrally of the tool 100. The electric motor 17 drives the converter element 3 to rotate continually in a single direction (as shown by arrow 11) at a controllable speed.

The converter element 3 comprises a cylindrical body having a first 19A and a second 19B groove extending around its circumference. Each groove 19A, 19B comprises a single continuous loop, extending within a plane at an angle to a radial plane through the body of the converter element 3. Each groove 19A, 19B is inclined at the same angle, but in opposite directions/senses. Thus, at a first point on the circumference of the converter element 3, the grooves 19A, 19B are relatively close together, but they diverge around the circumference from the first point, until at a second point diametrically opposite to the first they are relatively remote, each from the other. Continuing around the circumference from the second point, the grooves 19A, 19B converge back again towards the first point. The grooves 19A,19B thus each undergo a lateral displacement x, as measured along the longitudinal axis of the converter element 3 and the tool 100 as a whole. (See Figure 2 for a view of the converter element 3 in isolation).The blade driving cylinder 5A extends around a distal portion of the converter element 3, and is coupled to the converter element 3 by means of a driving stud 12 travelling within the first groove 19A.

A counterweight cylinder 5B extends coaxially around the gearbox 9 and a proximal portion of the converter element 3 and is coupled to the converter element by means of a driving stud 12 travelling within the second groove 19B.

As shown in Figure 1B, each driving stud 12 comprises a locating screw 16 extending into a metal, bush 18 within a high-density polyethylene (HDPE) block 14. As shown in Figure 1C, the locating screw 16 fastens the driving stud 12 to the blade driving cylinder 5A or the counterweight cylinder 5B, respectively, with the low-friction HDPE block 14 located within the respective first 19A or second groove 19B.

Thus, as the converter element 3 is rotated, the respective driving studs 12 must follow their respective grooves 19A, 19B (NB: there are spline arrangements, omitted for clarity, to prevent the cylinders 5A, 5B merely rotating along with the converter element 3). The driving studs 12 and their respective cylinders 5A, 5B are thus compelled to travel axially of the tool 100, first outwardly towards the remote ends of the tool 100 and then back towards each other. Because of the opposite inclination of the grooves 19A, 19B, the cylinders 5A, 5B thus move 180° out-of-phase (i.e in antiphase).

The blade driving cylinder 5A is mounted securely to the housing 10, the enclosed ultrasonic transducer 8 and the blade portion 2 of the tool 100. Thus, the entire acoustic system 1 is displaced reciprocally along the longitudinal axis of the tool 100, in particular producing a reciprocal longitudinal motion of the cutting head 6.

The particular tool 100 shown is set up for this reciprocal/oscillatory motion to be at a frequency of about 50Hz, with the lateral displacement x of the groove 19A, the blade driving cylinder 5A and the cutting head 6 being of the order of three to ten millimetres.

The counterweight cylinder 5B is constructed to have a mass as close as possible to the total mass of the blade driving cylinder 5A and the acoustic system 1, including the housing 10 and the blade portion 2. Thus, as the converter element 3 rotates and the counterweight cylinder 5B is also displaced with the same lateral displacement x at the same reciprocal/oscillatory frequency, a centre of mass of the counterweight cylinder 5B, blade driving cylinder 5A and acoustic system 1 should remain substantially stationary. Whereas a convertional vibrating saw at a frequency of around 50Hz would tend to give rise to vibrations transmitted into a user's hand (possibly causing discomfort, fatigue and even tissue damage after prolonged exposure), the tool 100 shown should produce minimal or zero tangible vibrations in the user's hand. This should allow longer periods of use and greater accuracy in use, since the user's hand should avoid fatigue for longer.

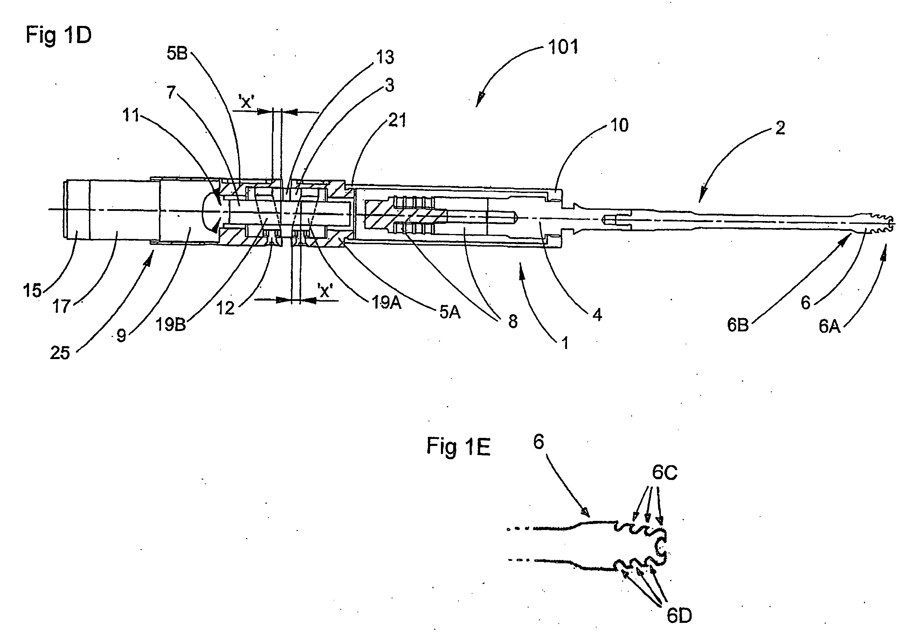

A second bone resector tool 101, shown in Figure 1D, is very similar to the first bone resector tool 100. Its longitudinal mode ultrasonic transducer 8, horn 4 and blade portion 2 are shown in more detail, as are the arrangements used to fasten the ultrasonic transducer 8, horn 4 and blade portion 2 together. The second tool 101 operates in an identical manner to the first tool 100.

The cutting head 6 of the second tool 101 is also shown in more detail in Figure 1D, and in particular in Figure IE. The cutting head 6 of the second tool 101 has two lateral cutting edges, which converge slightly towards its distal tip 6A. Each cutting edge is provided with an array of cutting teeth 6C. Each cutting tooth 6C has a hooked or "shark-tooth" profile, with a pointed tip of each hooked tooth aligned towards the distal tip 6A of the cutting head 6. The cutting teeth 6C are defined by an array of slanting notches 6D, each notch having an inner end with a profile comprising a portion of a circle.

While this form of cutting head 6 is of particular benefit when incorporated into bone resector tools 100, 101 as described above, it is believed that it would also be of benefit in other bone resector tools (osteotomes), particularly those in which the cutting head 6 is ultrasonically vibratable.

The converter element 3 is shown in more detail in Figure 2. The grooves 19A, 19B are as described above. Not shown above was an axial bore or passage 23, which receives a driveshaft 24 as shown in Figure 3. The cylindrical shaft 26 of the driveshaft 24 is provided with a flat 27. A radial aperture 13A extending through the converter element 3 into its axial bore 23 (Figure 2) allows a radial screw 13 (Figure 1) to engage with the flat 27 to secure the driveshaft 24 to the converter element 3. A proximal fitting 28 of the driveshaft 24 allows it to be connected to the gearbox 9.

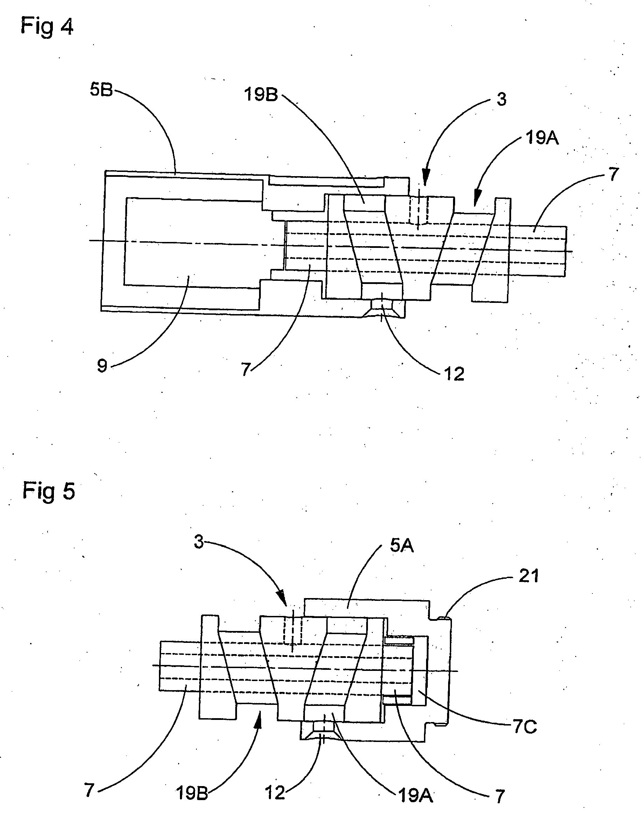

Figure 4 shows the counterweight cylinder 5B coupled to the converter element 3 by its driving stud 12 following the second groove 19B. In the disposition shown, the counterweight cylinder 5B is at its maximum displacement towards the centre of the tool 100.

In contrast, Figure 5 shows the blade driving cylinder 5A coupled to the converter element 3, but in a disposition in which the blade driving cylinder 5A is at its maximum displacement towards a distal end of the tool 100, 101. (Note the gap 7C between a distal end of the converter element 3 and the blade driving cylinder 3.

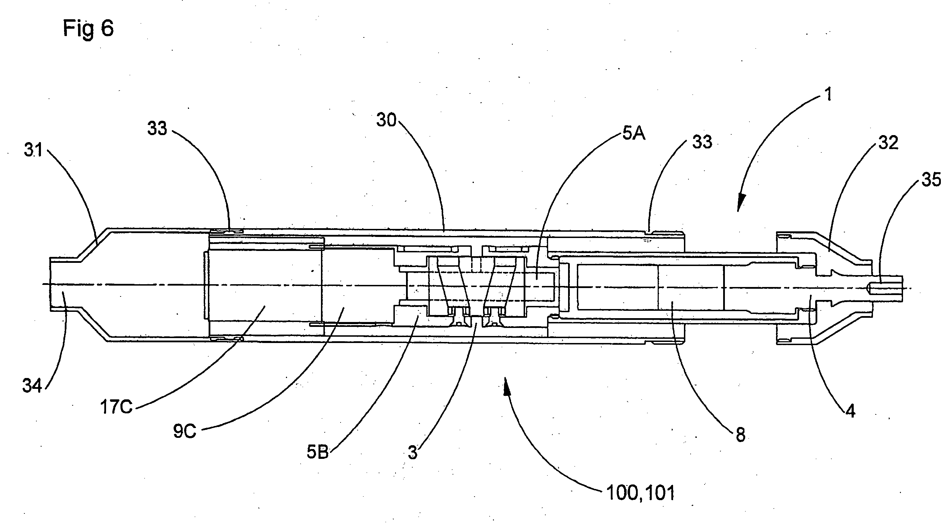

Figure 6 shows additional features of the tool 100, 101 as a whole. The internal operataive structures shown in Figure 1 are enclosed in a three-piece casing 30, 31, 32. A proximal cap 31 and a distal cap 32 are both detachably mounted to a main casing 30, with seals 33 provided at the respective joints to protect the internal workings of the tool 100, eg. from fluid ingress.

The main casing 30 encloses respective spaces 17C, 9C to hold the motor 17 and gearbox 9 (not shown), the converter element 3, both cylinders 5A, 5B and a proximal portion of the ultrasonic generator 8.

The proximal cap 31 has an opening 34 for power cables and control cables (it is common for such tools to be activated by means of a foot pedal, rather than by a finger-operated switch on the tool itself).

The detachable distal cap 32 allows access to the ultrasonic generator 8.

A further feature of this tool 100, 101 is that the blade portion 2 is detachable, using a threaded fitting 35. Blades having alternative cutting head 6 geometries may thus be fitted, and worn or damaged cutting heads 6 may be exchanged.

The tool 100, 101 shown thus have a cutting edge that is both vibrated ultrasonically and displaced reciprocally on a macroscopic scale at a much lower frequency. Combining ultrasonic activation and macroscopic blade reciprocation in this way creates a significant advantage in cutting efficiency. With sufficient ultrasonic amplitude, the physical force required to cut the bone is reduced to close to zero, while the reciprocating action displaces embrittled bone tissue with very little reactive force. This creates a vibration-free sensation as a surgeon cuts into the bone, with clear benefits for accuracy, comfort and reduced fatigue. The counterbalanced macroscopic reciprocating drive mechanism described above further enhances this substantially vibration-free action.

High amplitude ultrasound on its own heats the tissue on which it acts. Rapid and efficient removal of each layer of heated tissue by the macroscopic blade displacement avoids the bone necrosis that would otherwise be produced as this heat is dissipated into surrounding tissues.

This mechanism has been shown in animal model studies to produce an effective and safe method of bone resection. The studies indicated very low levels of bone necrosis, even without the saline irrigation that is conventionally employed for cleaning and cooling the cut site. Soft tissue disruption was negligible.

To gain maximum benefit in comfort and efficiency for the system shown, it has been found that the ultrasonic velocity amplitude should exceed the low frequency macroscopic velocity amplitude, preferably be a factor of between three and seven times. This ensures that the relative oscillatory movement of the cutting edge against bone tissue benefits substantially from friction vector reversal continuously throughout almost the entire cutting cycle of the reciprocating blade.

It should be appreciated that (regardless of frequency) holding a vibrating blade against tissue will produce a net heating effect. Only by moving the blade progressively through the target tissue can cutting be effected and heated tissue removed from the immediate surgical site. Manually-impelled bodily movement of the blade is impractical within the parameters described, so the combined action of the present invention has major practical benefits.

## Claims

1. A surgical tool (100, 101) adapted to cut osseous material, said tool (100, 101) comprising cutting head means (6) having cutting edge means, said cutting head means (6) being operatively connected to means to generate ultrasonic vibrations (8), and also being operatively connected to means (3, 17) to displace the cutting head means (6) reciprocally, **characterised in that** the means (3, 17) to displace the cutting head means (6) reciprocally are provided with first counterweight means (5B) for the cutting head means (6), said first counterweight means (5B) being reciprocally displaceable out of phase with the cutting head means (6).

2. A surgical tool as claimed in claim 1, **characterised in that** the reciprocal displacement means (3, 17) displaces the cutting head means (6) generally parallelly to the cutting edge means.

3. A surgical tool as claimed in either claim 1 or claim 2, **characterised in that** the first counterweight means (5B) is reciprocally displaceable substantially in antiphase with the cutting head means (6).

4. A surgical tool as claimed in any one of the preceding claims, **characterised in that** a centre of mass of the cutting head means (6) and the first counterweight means (5B) remains substantially stationary.

5. A surgical tool as claimed in claim 1, **characterised in that** the means (3, 17) to displace the cutting head means (6) reciprocally displaces both the cutting head means (6) and the means to generate ultrasonic vibrations (8).

6. A surgical tool as claimed in claim 5, **characterised in that** the reciprocal displacement means (3, 17) is provided with second counterweight means (5B) for both the cutting head means (6) and the means to generate ultrasonic vibrations (8), reciprocally displaceable out of phase therewith.

7. A surgical tool as claimed in claim 5, **characterised in that** the reciprocal displacement means (3, 17) is provided with second counterweight means (5B) comprising said first counterweight means, said second counterweight means (5B) being counterweight means for both the cutting head means (6) and the means to generate ultrasonic vibrations (8), and being reciprocally displaceable out of phase therewith.

8. A surgical tool as claimed in either claim 6 or claim 7, **characterised in that** the second counterweight means (5B) is reciprocally displaceable substantially in antiphase with the cutting head means (6) and the means to generate ultrasonic vibrations (8).

9. A surgical tool as claimed in any one of claims 6 to 8, **characterised in that** a centre of mass of the cutting head means (6), the means to generate ultrasonic vibrations (8) and the second counterweight means (5B) remains substantially stationary.

10. A surgical tool as claimed in any one of the preceding claims, **characterised in that** the reciprocal displacement means (3, 17) comprises a rotatable generally cylindrical body (3) having a first track means (19A) and a second track means (19B) each extending continuously around the body (3), with the cutting head means (6) and optionally the means to generate ultrasonic vibrations (8) being moveably engaged with the first track means (19A), and the respective counterweight means (5B) being moveably engaged with the second track means (19B).

11. A surgical tool as claimed in claim 10, **characterised in that** each track means (19A, 19B) extends around the rotatable cylindrical body (3) at an angle to a rotational axis of the body (3), with the first track means (19A) being angled in an opposite sense to the second track means (19B).

12. A surgical tool as claimed in any one of the preceding claims, **characterised in that** the relative amplitudes of the ultrasonic vibrations and the reciprocal displacement of the cutting head means (6) are such that a peak velocity of the cutting head means (6) due to the ultrasonic vibrations is greater than a peak velocity resulting from the reciprocal displacement of the cutting head means (6).

13. A surgical tool as claimed in claim 12, **characterised in that** the peak velocity of the cutting head means (6) due to the ultrasonic vibrations is at least twice and no more than ten times the peak velocity resulting from the reciprocal displacement of the cutting head means (6).

14. A surgical tool as claimed in any one of the preceding claims, **characterised in that** the reciprocal displacement means (3, 17) is adapted to produce a reciprocal oscillatory motion at a frequency of 250Hz or lower, and optionally at a frequency of at least 20Hz.

15. A surgical tool as claimed in any one of the preceding claims, **characterised in that** the cutting edge means is provided with a plurality of hooked tooth means (6C), optionally with a tip of each hooked tooth means (6C) extending generally towards a distal tip (6A) of the tool (101).

## Patentansprüche

1. Chirurgisches Werkzeug (100, 101), das zum Schneiden von Knochenmaterial eingerichtet ist, wobei das Werkzeug (100, 101) Schneidkopfmittel (6) umfasst, die Schneidkantenmittel aufweisen, wobei die Schneidkopfmittel (6) wirksam mit Mitteln (8) zum Erzeugen von Ultraschallvibrationen verbunden sind und ebenfalls wirksam mit Mitteln (3, 17) zum hin- und hergehenden Verschieben der Schneidkopfmittel (6) verbunden sind, **dadurch gekennzeichnet, dass** die Mittel (3, 17) zum hin- und hergehenden Verschieben der Schneidkopfmittel (6) mit ersten Gegengewichtsmitteln (5B) für die Schneidkopfmittel (6) versehen sind, wobei die ersten Gegengewichtsmitteln (5B) außer Phase mit den Schneidkopfmitteln (6) hin- und hergehend verschiebbar sind.

2. Chirurgisches Werkzeug nach Anspruch 1, **dadurch gekennzeichnet, dass** die hin-und hergehenden Verschiebungsmittel (3, 17) die Schneidkopfmittel (6) im Wesentlichen parallel zu den Schneidkantenmitteln verschieben.

3. Chirurgisches Werkzeug nach Anspruch 1 oder Anspruch 2, **dadurch gekennzeichnet, dass** die ersten Gegengewichtsmittel (5B) hin- und hergehend im Wesentlichen in Gegenphase mit den Schneidkopfmitteln (6) verschiebbar sind.

4. Chirurgisches Werkzeug nach einem der vorhergehenden Ansprüche, **dadurch gekennzeichnet, dass** ein Massenschwerpunkt der Schneidkopfmittel (6) und der ersten Gegengewichtsmittel (5B) im Wesentlichen unbeweglich bleibt.

5. Chirurgisches Werkzeug nach Anspruch 1, **dadurch gekennzeichnet, dass** die Mittel (3, 17) zum hin- und hergehenden Verschieben der Schneidkopfmittel (6) sowohl die Schneidkopfmittel (6) als auch die Mittel (8) zum Erzeugen von Ultraschallvibrationen hin- und hergehend verschieben.

6. Chirurgisches Werkzeug nach Anspruch 5, **dadurch gekennzeichnet, dass** die hin-und hergehenden Verschiebungsmittel (3, 17) mit zweiten Gegengewichtsmitteln (5B) für sowohl die Schneidkopfmittel (6) als auch die Mittel (8) zum Erzeugen von Ultraschallvibrationen, die außer Phase mit denselben hin- und hergehend verschiebbar sind, versehen sind.

7. Chirurgisches Werkzeug nach Anspruch 5, **dadurch gekennzeichnet, dass** die hin-und hergehenden Verschiebungsmittel (3, 17) mit zweiten Gegengewichtsmitteln (5B) versehen sind, welche die ersten Gegengewichtsmittel einschließen, wobei die zweiten Gegengewichtsmittel (5B) Gegengewichtsmittel sowohl für die Schneidkopfmittel (6) als auch die Mittel (8) zum Erzeugen von Ultraschallvibrationen sind und außer Phase mit denselben hin- und hergehend verschiebbar sind.

8. Chirurgisches Werkzeug nach Anspruch 6 oder Anspruch 7, **dadurch gekennzeichnet, dass** die zweiten Gegengewichtsmittel (5B) im Wesentlichen in Gegenphase mit den Schneidkopfmitteln (6) und den Mitteln (8) zum Erzeugen von Ultraschallvibrationen hin- und hergehend verschiebbar sind.

9. Chirurgisches Werkzeug nach einem der Ansprüche 6 bis 8, **dadurch gekennzeichnet, dass** ein Massenschwerpunkt der Schneidkopfmittel (6), der Mittel (8) zum Erzeugen von Ultraschallvibrationen und der zweiten Gegengewichtsmittel (5B) im Wesentlichen unbeweglich bleibt.

10. Chirurgisches Werkzeug nach einem der vorhergehenden Ansprüche, **dadurch gekennzeichnet, dass** die hin- und hergehenden Verschiebungsmittel (3, 17) einen drehbaren, im Wesentlichen zylindrischen Körper (3) umfasst, der ein erstes Spurmittel (19A) und ein zweites Spurmittel (19B) aufweist, die sich jeweils durchgehend um den Körper (3) erstrecken, wobei die Schneidkopfmittel (6) und wahlweise die Mittel (8) zum Erzeugen von Ultraschallvibrationen beweglich mit dem ersten Spurmittel (19A) in Eingriff gebracht sind und die jeweiligen Gegengewichtsmittel (5B) beweglich mit dem zweiten Spurmittel (19B) in Eingriff gebracht sind.

11. Chirurgisches Werkzeug nach Anspruch 10, **dadurch gekennzeichnet, dass** sich jedes Spurmittel (19A, 19B) in einem Winkel zu einer Drehachse des Körpers (3) um den drehbaren zylindrischen Körper (3) erstreckt, wobei das erste Spurmittel (19A) in einem entgegengesetzten Sinn zu dem zweiten Spurmittel (19B) abgewinkelt ist.

12. Chirurgisches Werkzeug nach einem der vorhergehenden Ansprüche, **dadurch gekennzeichnet, dass** die verhältnismäßigen Amplituden der Ultraschallvibrationen und der hin- und hergehenden Verschiebung der Schneidkopfmittel (6) derart sind, dass eine Spitzengeschwindigkeit der Schneidkopfmittel (6) auf Grund der Ultraschallvibrationen größer ist als eine Spitzengeschwindigkeit, die sich aus der hin- und hergehenden Verschiebung der Schneidkopfmittel (6) ergibt.

13. Chirurgisches Werkzeug nach Anspruch 12, **dadurch gekennzeichnet, dass** die Spitzengeschwindigkeit der Schneidkopfmittel (6) auf Grund der Ultraschallvibrationen wenigstens das Zweifache und nicht mehr als das Zehnfache der Spitzengeschwindigkeit, die sich aus der hin- und hergehenden Verschiebung der Schneidkopfmittel (6) ergibt, beträgt.

14. Chirurgisches Werkzeug nach einem der vorhergehenden Ansprüche, **dadurch gekennzeichnet, dass** die hin- und hergehenden Verschiebungsmittel (3, 17) dafür eingerichtet sind, eine hin- und hergehende oszillierende Bewegung mit einer Frequenz von 250 Hz oder weniger und wahlweise mit einer Frequenz von wenigstens 20 Hz zu erzeugen.

15. Chirurgisches Werkzeug nach einem der vorhergehenden Ansprüche, **dadurch gekennzeichnet, dass** die Schneidkantenmittel mit mehreren Hakenzahnmitteln (6C) versehen sind, wobei sich wahlweise eine Spitze jedes Hakenzahnmittels (6C) im Allgemeinen zu einer distalen Spitze (6A) des Werkzeugs (101) hin erstreckt.

## Revendications

1. Outil chirurgical (100, 101), adapté pour couper du matériau osseux, ledit outil (100, 101) comprenant un moyen de tête de coupe (6) comportant un moyen d'arête de coupe, ledit moyen de tête de coupe (6) étant connecté en service à un moyen destiné à générer des vibrations ultrasonores (8) et étant également connecté en service à un moyen (3, 17) destiné à déplacer le moyen de tête de coupe (6) de manière réciproque, **caractérisé en ce que** le moyen (3, 17) de déplacement réciproque du moyen de tête de coupe (6) comporte un premier moyen de contrepoids (5B) pour le moyen de tête de coupe (6), ledit premier moyen de contrepoids (5B) pouvant être déplacé de manière réciproque hors de phase avec le moyen de tête de coupe (6).

2. Outil chirurgical selon la revendication 1, **caractérisé en ce que** le moyen de déplacement (3, 17) déplace le moyen de tête de coupe (6) en général de manière parallèle au moyen d'arête de coupe.

3. Outil chirurgical selon les revendications 1 ou 2, **caractérisé en ce que** le premier moyen de contrepoids (5B) peut être déplacé de manière réciproque, substantiellement en antiphase avec le moyen de tête de coupe (6).

4. Outil chirurgical selon l'une quelconque des revendications précédentes, **caractérisé en ce qu'**un centre de masse du moyen de tête de coupe (6) et du premier moyen de contrepoids (5B) reste sensiblement stationnaire.

5. Outil chirurgical selon la revendication 1, **caractérisé en ce que** le moyen (3, 17) de déplacement réciproque du moyen de tête de coupe (6) déplace de manière réciproque le moyen de tête de coupe (6) et le moyen de génération des vibrations ultrasonores (8).

6. Outil chirurgical selon la revendication 5, **caractérisé en ce que** le moyen de déplacement réciproque (3, 17) comporte un deuxième moyen de contrepoids (5B) pour le moyen de tête de coupe (6) et le moyen de génération des vibrations ultrasonores (8), pouvant être déplacé de manière réciproque hors de phase avec ceux-ci.

7. Outil chirurgical selon la revendication 5, **caractérisé en ce que** le moyen de déplacement réciproque (3, 17) comporte un deuxième moyen de contrepoids (5B) comprenant ledit premier moyen de contrepoids, ledit deuxième moyen de contrepoids (5B) étant un moyen de contrepoids pour le moyen de tête de coupe (6) et le moyen de génération de vibrations ultrasonores (8) et pouvant être déplacé de manière réciproque hors de phase avec ceux-ci.

8. Outil chirurgical selon les revendications 6 ou 7, **caractérisé en ce que** le deuxième moyen de contrepoids (5B) peut être déplacé de manière réciproque, sensiblement en antiphase avec le moyen de tête de coupe (6) et le moyen de génération de vibrations ultrasonores (8).

9. Outil chirurgical selon l'une quelconque des revendications 6 à 8, **caractérisé en ce qu'**un centre de masse du moyen de tête de coupe (6), du moyen de génération de vibrations ultrasonores (8) et du deuxième moyen de contrepoids (5B) reste sensiblement stationnaire.

10. Outil chirurgical selon l'une quelconque des revendications précédentes, **caractérisé en ce que** le moyen de déplacement réciproque (3, 17) comprend un corps rotatif généralement cylindrique (3), comportant un premier moyen de piste (19A) et un deuxième moyen de piste (19B), s'étendant chacun de manière continue autour du corps (3), le moyen de tête de coupe (6) et optionnellement le moyen de génération des vibrations ultrasonores (5B) étant engagés de manière mobile dans le premier moyen de piste (19A) et le moyen de contrepoids respectif (5B) étant engagé de manière mobile dans le deuxième moyen de piste (19B).

11. Outil chirurgical selon la revendication 10, **caractérisé en ce que** chaque moyen de piste (19A, 19B) s'étend autour du corps rotatif cylindrique (3) à un angle par rapport à un axe de rotation du corps (3), le premier moyen de piste (19A) étant incliné dans un sens opposé par rapport au deuxième moyen de piste (19B).

12. Outil chirurgical selon l'une quelconque des revendications précédentes, **caractérisé en ce que** les amplitudes relatives des vibrations ultrasonores et du déplacement réciproque du moyen de tête de coupe (6) sont tels qu'une vitesse de pointe du moyen de tête de coupe (6), due aux vibrations ultrasonores, est supérieure à une vitesse de pointe résultant du déplacement réciproque du moyen de tête de coupe (6).

13. Outil chirurgical selon la revendication 12, **caractérisé en ce que** la vitesse de pointe du moyen de tête de coupe (6) due aux vibrations ultrasonores représente au moins deux fois et pas plus de dix fois la vitesse de pointe résultant du déplacement réciproque du moyen de tête de coupe (6).

14. Outil chirurgical selon l'une quelconque des revendications précédentes, **caractérisé en ce que** le moyen de déplacement réciproque (3, 17) est adapté pour produire un mouvement réciproque oscillatoire à une fréquence de 250 Hz ou moins, et optionnellement à une fréquence d'au moins 20 Hz.

15. Outil chirurgical selon l'une quelconque des revendications précédentes, **caractérisé en ce que** le moyen d'arête de coupe comporte plusieurs moyens de dents à crochet (6C), une pointe de chaque moyen de dent à crochet (6C) s'étendant optionnellement en général vers une pointe distale (6A) de l'outil (101).
